# EUROPEAN PATENT APPLICATION

(11) **EP 3 753 516 A1**
(43) Date of publication of application: **23.12.2020**
(21) Application number: 20179728.9
(22) Date of filing: 12.06.2020
(51) Int. Cl.: A61B 18/12, A61N 1/32

(54) **ELECTROPORATION DEVICE**

(30) Priority: 21.06.2019 CZ 20190400
(71) Applicant: FAKULTNI NEMOCNICE U SV. ANNY V BRNE, 60200 Brno (CZ); Vysoké Uceni Technické V Brne, 60200 Brno (CZ)
(72) Inventor: Starek, Zdenek, 63600 Brno (CZ); Pesl, Martin, 60200 Brno (CZ); Wolf, Jiri, 68738 Nedakonice (CZ); Caluori, Guido, 60200 Brno (CZ); Cervinka, Dalibor, 66451 Kobylnice (CZ); Martis, Jan, 66401 Ricmanice (CZ); Novotna, Veronika, 69165 Krepice (CZ)
(74) Representative: Benda, Tomas

(57) **Abstract**

Alternating electroporation generator, where it contains control unit (8) connected with regulation unit (9), direct current power supply (10), and power converter (11), containing storage capacitor and four power transistors of the type MOSFET, connected into H-bridge, in whose diagonal is connected primary winding of the output high-frequency pulse transformer (12) on ferrite core, whose secondary winding is connected with at least one application electrode.

## Description

### Field of invention

The present invention relates to alternating electroporation generator, i.e. source of high voltage pulses for the purpose of electroporation.

### Background of the invention

Catheter ablation of cardiac arrhythmia is a standard therapeutic approach for patients suffering from heart rhythm disorders resistant to medication. The principal of this approach is generation of heat around the electrode that is in contact with conductive tissue where temperature increase induces immediate and at the same time localized cellular necrosis. Electrically non-conductive scar then forms in the process of healing that prevents pathological passing of the action potential.

The most common technique is Radiofrequency Ablation abbreviated as "RFA", whose advantages are in respect to nature of the cure limited by risks and limited efficiency. Examples of the risks comprise damage of surrounding structures, e.g. esophagus, phrenic nerve or coronary vessels, and formation of so called "pops", rapidly forming expansion of vapor arising from overheating of the myocardium in the place of contact of the catheter apex with tissue resulting in damage of the myocardium with the possibility of rupture of the heart wall. The limits of efficiency are in relatively frequent recurrence of arrhythmia that is due to restoring the conduction of action potential during healing of ablation lesions, which is caused by insufficient dimension and depth of ablation lesions.

A convenient alternative overcoming above mentioned limits are non-thermal methods of ablations, using which may avoid complications typical for standard ablation therapy. Technique of irreversible electroporation, abbrev. "IRE" is known for more than thirty years and presently is used for liquidation of some kinds of cancer. The electroporation may be generally induced by direct current (DC) and alternating current (AC) high voltage pulses.

Currently, generators with high performance exist that enable to ensure sufficient intensity of electric field for induction of formation of pores in cell membranes inside tissue for sufficiently long period needed for induction of controlled remodelation and scar formation used in oncotherapy. Generators with high performance that had been intended for specific therapy of cardiac arrhythmia, are so far commercially unavailable. Direct use of existing generators DC IRE in the field of cardiology is not possible due to a number of potential risks. A serious risk is represented by generation of electrolytic gas, during application of DC IRE using direct current, in the form of bubbles with consequent risk of embolization into the brain. At the same time, cell necrosis might occur as a consequence of pH changes in the area of application of DC IRE. The effect of DC IRE pulses induces life threatening ventricular fibrillation. DC pulses trigger contractions of skeletal muscles, which is very unpleasant and painful for the patient and the procedure using DC IRE must be performed in total anesthesia, which complicates the ablation procedure and causes its cost rise. A solution might be shortening of the length of the pulses or decrease of voltage value, nevertheless, the efficiency of the therapy is thus reduced. Reduction in the effect might be compensated by lengthening the period of application (increase of the number of applied pulses). That is, however, possible with current IRE generators only in narrow range. It has been, however, experimentally proved that application of any DC pulses in the area of myocardium is risky.

In the case of applied sequence of AC pulses, the undesirable heart reaction is significantly lower. Also, in the case of fault in synchronization of application of AC pulse and ECG signal and at pulse delivery in the least convenient moment is reaction detectable on ECG signal but it does not lead to life threatening ventricular fibrillation. This circumstance significantly increases the safety of the procedure and decreases the demands on accuracy and reliability of synchronization with ECG signal versus DC IRE.

The first commercially available device for ablation using DC IRE has been since 2009 device NanoKnife of US company AngioDynamics. NanoKnife is approved generally for surgery ablation of soft tissues, however, not for therapy of a specific diagnosis. The device uses pulses of high DC voltage of low energy in bipolar configuration, i.e. electric current passes between two electrodes immersed into ablated tissue. DC electric field that arises between the application electrodes induces formation of nanopores in cellular membrane. Thus, the cell homeostasis is disrupted, and the cells die by cell death. NanoKnife enables using of up to six electrodes, but at least two. A disadvantage of this device is use of DC current. As is stated above, it is necessary that the patient is in total anesthesia and moreover muscle relaxants must be administered, so as undesirable spams of skeletal muscles and related body movements do not occur during the procedure. Further, it is necessary that the application pulses are synchronized with ECG signal, so that application of pulse in vulnerable phase of chamber cycle, which would lead to fibrillation, is avoided.

Similar functions as system NanoKnife are provided by experimental DC electroporation generator published in article "High-Voltage Pulse Source for Cell Electroporation", in Mechatronics 2017. Like NanoKnife, it generates tens of microseconds long pulses of DC high voltage. Desired function is reached by using large transformer with core of iron sheets, whose primary winding is powered by single acting open-loop converter from transistors IGBT and secondary winding is connected to diode rectifier. From the view of practical application, this solution, however, provides identical disadvantage that was described above and that is given in principal by using DC voltage.

This generator is, however, inappropriate for application in cardiology because of inadmissible muscle contractions that are caused by application of pulses of DC voltage.

In the following years, the use of alternating voltage has begun to be considered, where there is no need of anesthesia, muscle relaxation and eventually also synchronization with ECG signal. Patent document US 2010/0023004 relates to system and method for ablation of cardiac tissue using electroporation. The generator provides voltage on the output of 200 - 700 V and forms pulses of length 50 -200 µs, with spaces between the pulses of length 100-400 µs. These pulses and spaces form aggregates. The aggregates are of length 200 - 1000 ms and spaces between the aggregates are of length about 1 s. Together, it forms sequence of length 2 - 6 s. A disadvantage, however, is, that the application electrodes are connected directly to output of transistor H-bridge. In the case of failure of the transistor or control circuits, full DC power voltage on the application electrode of 750 V may occur, which is dangerous for the patient and inadmissible for a medical device.

Patent application US 2012/0310237 A1 relates to systems and methods for high voltage ablation. Specifically, for least invasive therapy of atrial fibrillation using bipolar pliers with electrodes and monopolar applicator. It relates to rather an applicator of pulses than system that supplies pulses. Maximal applied voltage in one configuration is 3000 V. The mention of system using alternating voltage is only in the sense of potential protection against ventricular tachycardia or fibrillation at application of high-voltage pulses.

Patent application US 2015/0201991 A1 relates to ablation of myocardium using nanosecond electric pulse fields. The device is intended for treatment of atrial and ventricular arrhythmia. Voltage pulses are of length 1 - 1000 ns with amplitude 1 - 100 kV. The document does not specify the device producing pulses of such extreme parameters and neither reflects a number of safety risks.

Patent application US 2016/0331441 A1 describes asymmetric waveform for short sequences of alternating current AC irreversible electroporation on heart. It states, that this type of wave may be used for AC as well as for DC electroporation. The subject of the document is electroporation generator that forms upon its output a sequence made of positive and negative current pulses. The sequence is not described in detail and neither is the form of asymmetry wave specified numerically. It is described so that it is made of first positive phase and first negative phase. Each of these phases has given current and time so that the first current is larger than the second and the second time is larger than the first. This asymmetric form is configured for irreversible electroporation in target tissue. The text of the document does not imply that the forms have high-frequency character, i.e. in hundreds of kHz.

Patent application US 2017/0035499 A1 relates to cardiac pulse ablation. Described here is a method that comprises administration of a series of bipolar pulses of at least 60 pulses. Each bipolar pulse is formed by one positive and one negative pulse, where each of them has 1 - 15 µs and voltage in the range 300 - 4000 V. The negative pulse follows the positive pulse either immediately or within 5 µs. The spaces between individual pulses are 5 - 800 µs long. A pulse is applied 70 - 100 ms after R wave of ECG signal. The device thus enables also analysis of ECG signal. A change in pH at the interphase between electrode and tissue caused by electrolysis may, however, occur at application of such long sequence of pulses.

The most recent contribution in this field is the article "Ablation of atrial fibrillation with pulsed electric fields: An ultra-rapid, tissue-selective modality for cardiac ablation", where the authors performed a clinical study of new endocardial and epicardial ablation system using pulse electric field, abb. "PEF". The system for this ablation consists of a single-purpose generator of high-voltage pulses and catheters. The first catheter contains five sectors, where each has four separated electrodes for pulse application. This catheter is for endocardial ablation. The second catheter used for epicardial ablation has a form of elastic loop with electrodes, where always two electrodes are active and take turns cyclically. The generator produces unspecified bipolar millisecond pulses. The only adjustable parameter of the pulses is their voltage in the range 900 - 2500 V, the other properties of the pulses are fixed. A disadvantage is, however, the necessity of total anesthesia and muscle relaxation.

The target of the present invention is to introduce easily feasible and safe electroporation generator that eliminates above mentioned shortcomings of the state of art.

### Summary of the invention

Above mentioned shortcomings are to a large extent eliminated by the alternating electroporation generator, whose principal lies in that it contains a control unit connected with regulation unit, DC power supply, power converter, containing storage capacitor and four power transistors of type MOSFET, connected into H-bridge, in whose diagonal is connected primary winding of input high-frequency pulse transformer on ferrite core, whose secondary winding is connected with at least one application electrode.

In another preferred embodiment, there is one or two application electrodes.

In another preferred embodiment, the application electrodes are two catheters with one contact area, or at least one catheter with more contact areas, or a catheter in combination with a large-area contact electrode for placement in contact with body surface of the patient.

In another embodiment is the pulse transformer preferably in the form of changeable module allowing its change for another kind of transformer with different ratio of the magnitude of the output voltage and current.

In another embodiment is DC source powered by rectified mains voltage, whereas it is a changeable DC source of voltage that enables adjustment of magnitude of the voltage of output pulses.

In another preferred embodiment contains the alternating electroporation generator further current sensor on the output that enables, through display device, to display the course of the current of electroporation pulse brought into application electrodes.

In another preferred embodiment is the control unit designed to control the power converter, through which it enables setting of all parameters of thus formed pulse.

In another preferred embodiment, it further contains ECG sensor electrode connected with ECG monitor connected with block of ECG analysis and synchronization of pulses connected with control unit.

Above mentioned shortcomings are to a large measure eliminated also by use of alternating electroporation generator according to any of the claims for cardiology.

### Description of the drawings

The present invention will be further described in the figures, where Fig. 1 represents block schema of alternating electroporation generator according to the present invention, Fig. 2 represents block schema of one of the variants of alternating electroporation generator according to the present invention, Fig. 3 represents time course of the output voltage of alternating electroporation generator according to the present invention, Fig. 4 represents current response measured during application of alternating electroporation generator according to the present invention, Fig. 5 represents synchronization of application of the pulses AC IRE with ECG signal, Fig. 6 represents electrogram before AC IRE ablation, and Fig. 7 represents electrogram after AC IRE ablation.

### Example of realization of the invention

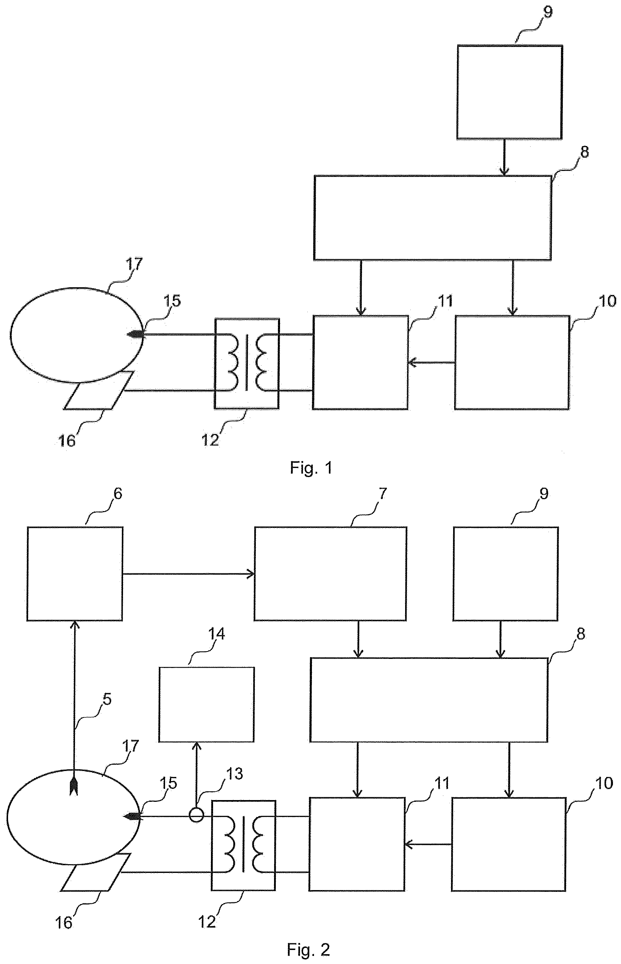

Alternating electroporation generator according to the present invention is displayed in Fig. 1 and contains control unit 8 connected with regulation unit 9, direct current power supply 10 and power converter 11, whereas converter 11 is connected with DC power supply 10 as well as with primary winding of isolation pulse transformer 12 with ferrite core, whose secondary winding is connected to application electrodes used for bringing electroporation pulse into tissue. That can be for example two catheters 15 with one contact area, or one or more catheters 15 with more contact areas, or catheter 15 in combination with a large-area contact electrode 16 for placing into contact with body surface of the patient 17.

Regulation unit 9 contains control elements for setting of pulse parameters such as voltage, frequency, pulse length, length of space between pulses and their number. It contains also displays, displaying these adjustable parameters.

In one preferred embodiment, the generator according to the invention presented in Fig. 2, contains ECG sensor electrode 5 connected with ECG monitor 6 connected with block 7 of analysis of ECG and synchronization of pulses connected with control unit 8.

ECG sensor electrode 5 is a standard sticking electrode using measurement amplifier or ECG monitor of common construction.

Block 7 of the ECG analysis and pulse synchronization may be realized using PC equipped with measurement cards. PC environment enables easy assessment of the course of ECG and determination of the proper moment for application of electroporation pulse.

Control unit 8 contains control and safety circuits that control power converter 11, and thus ensure realization of pulses of desired parameters. Control unit 8 is preferably connected with external ECG monitor, i.e. through block of ECG analysis and synchronization of pulses 7, to ensure function of synchronization of electroporation pulse with ECG signal. Control unit 8 is triggered with this block 7 of ECG analysis and synchronization of pulses. In the case that synchronization with ECG is not used, its control circuits work autonomously and produce pulses of constant period set on control panel.

Direct current power supply 10 is powered from the mains and it can be preferably a variable DC voltage source, thanks to which the setting of voltage amplitude of output pulses is enabled.

Power converter 11 contains storage capacitor and four power transistors, preferably of type MOSFET connected into H-bridge, in whose diagonal is connected primary winding of the output pulse transformer 12.

Output impulse transformer 12 ensures change of magnitude of the output voltage of the pulses to desired value, and galvanic separation of the output alternating voltage from a DC source. Its primary winding is connected to power converter 11 and secondary winding is connected to the output of alternating electroporation generator according to the present invention, e.g. into catheter 15 and contact electrodes 16. The transformer 12 winding has due to safety multiple isolation between primary and secondary part. This isolation layer may be formed by e.g. 1 mm thick layer of isolation plastic. The winding is wound onto ferrite core due to presence of high frequency. Thanks to the presence of this transformer 12, there is, in the case of transistor damage in power converter 11, excluded the possibility of permanent attachment of DC voltage to its output. In the event of short circuit or fault in control circuits leading to its permanent switching on, magnetization current of transformer 12 will grow within a few microsecond so that the transistors themselves irreversibly interrupt and shortly afterwards disconnect primary winding of transformer 12 from storage capacitor of the converter and direct current power supply 10. This property of alternating electroporation generator cannot be ensured in the case where transformer 12 is not present on the output.

Secondary winding of transformer 12 is connected to application electrodes serving application of the pulses into tissue. Configuration of application electrodes may be bipolar or unipolar. In the case of bipolar embodiment, the electrodes are formed e.g. by two catheters 15 or two or more contact areas contained in one catheter 15. In the case of unipolar embodiment of application electrodes is one application electrode formed e.g. by catheter 15 and large-area contact electrode 16 placed on the surface of the patient's 17 body.

Transformer 12 is made preferably in the form of changeable module enabling its change for different type, with embodiment and dimensions identical and of the same power but with different number of secondary turns. Thus, pulses of different ratio of current and voltage according to demands of given application electrode might be easily obtained by minimal service intervention into generator. Output alternating voltage will be e.g. 1.5-fold higher and current 1.5-fold lower. Power converter 11 powered from direct current power supply 10 is used to supply transformer 12.

As is obvious from Fig. 2, secondary winding of transformer 12 is preferably provided with current sensor 13 connected to display device 14. Current sensor 13 is galvanically separated and has large bandwidth. It allows to display the course of current of electroporation pulse into application electrodes through display device 14 represented e.g. by an oscilloscope. Since the amplitude of alternating voltage in the course of the pulse is almost constant, the quality of application electrode with tissue may be assessed from the size and shape of the current response and thus the success of the pulse application may be assessed.

Alternating electroporation generator according to the present invention generates pulses either autonomously or based on synchronization signal obtained by measurement and analysis of the ECG signals.

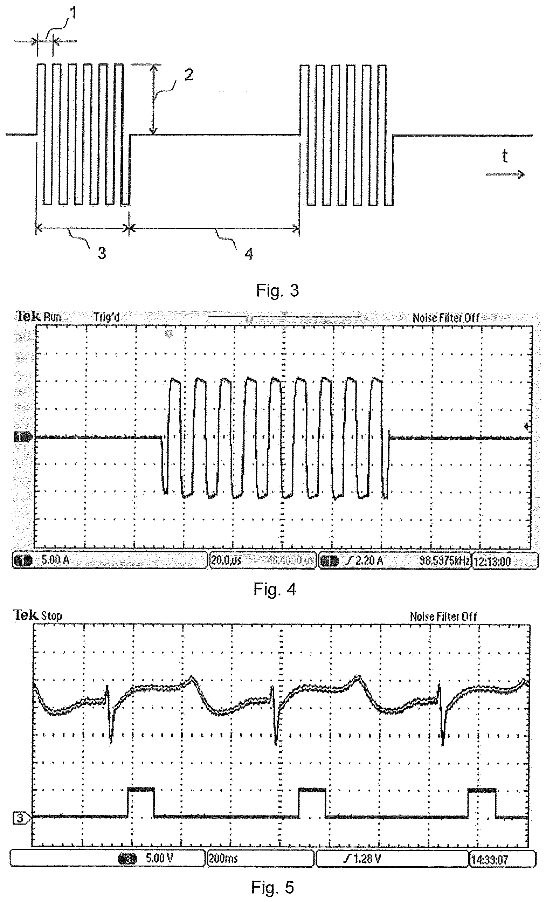

Tens to hundreds of microseconds long pulses of alternating voltage of frequency in hundreds kHz and of variable magnitude in the order of hundreds of volts, are usually applied by control unit 8 that are separated by spaces in tenths to units of seconds, see Fig. 3, where 1 is period of high frequency voltage, 2 is voltage amplitude, 3 is pulse duration, 4 is duration of space between pulses, t represents time.

Certain combination of voltage and current range is advantageous for different types of used application electrodes.

The preparation for application of pulses proceeds similarly to common radiofrequency catheter ablation, abbreviation "RFA".

Catheters 15 are introduced into heart cavity in local anesthesia through inguinal vein or optionally through inguinal artery.

Diagnostics of arrhythmia is performed after placing diagnostic catheters 15.

Ablation catheter 15 or optionally in the case of bipolar configuration catheters 15 are then placed in suitable ablation site, wherein the application AC IRE is later performed. Typical course of the pulse is shown in Fig. 4.

The contact between tissue and catheter 15 is verified using intracardiac ECG, or using fluoroscopy or anatomic 3D mapping system. Cardiac stimulation of stable frequency in the range 80 - 120 pulse/min allows to safely feel the pulse application outside the refractory period, that is within 150 ms of QRS complex.

Blocks 5 to 7 perform detection and analysis of ECG signal in real time. Shown in Fig. 5 is an AC IRE pulse synchronized with ECG signal. Average delay between QRS complex and application pulse is 75 ms. Rectangle signal is obtained from control LED.

Impedance between the apex of ablation catheter 15 and its reference is then measured before and after pulse application, which enables evaluation of its resulting effect.

A series of pulses is then triggered automatically and synchronized according to ECG following the set values. Tens to hundreds of pulses, e.g. 60, of width 10 - 150 µs, with spaces depending on heart rhythm or stimulation (pacing) and on frequency of alternating pulse 70 - 450 kHz are applied for best possible result. Effective range of pulse energy is in the order of tens J.

Current is measured during the pulses with catheter 15 using current sensor 13 and its value is displayed on display device 14. The efficacy of pulse application may be judged from displayed current course. Too small current signalizes interruption of catheter 15 wires.

Control impedance measurement with decrease of 15-20% is considered as mark of successful application, similarly to RFA. In the following time, the patient uses besides self-monitoring also continual 24h ECG Holter monitoring, or loop ECG recorders. As successful may be the ablation considered at absence of recurrence of arrhythmia after discontinuation of antiarrhythmic drugs after blind period, that is in RFA after cca 6-12 weeks.

Alternating electroporation generator according to the present invention is suitable for arrhythmology/cardiology application IRE because biphasic pulses corresponding to short frequencies of alternating current (AC), generated with special AC generator IRE, are more suitable than DC pulses. Moreover, no electrolysis and formation of bubbles occurs, the application of AC IRE does not induce ventricular fibrillation and the induction of contraction of skeletal muscles is lower than in the case of DC IRE.

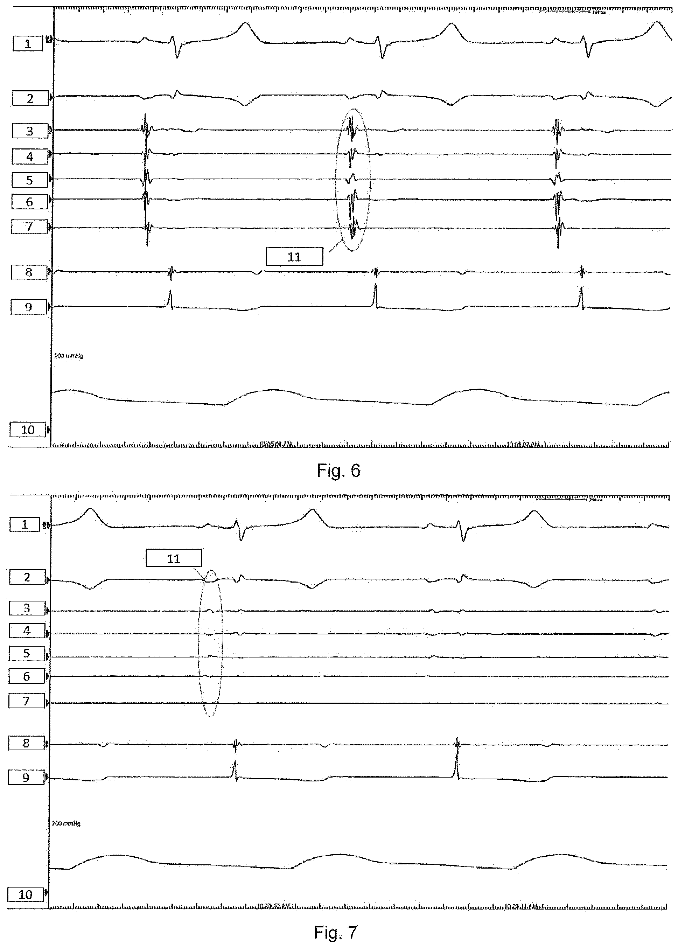

Alternating electroporation generator according to the present invention provides sufficient energy for efficient IRE ablation of myocardium, see Fig. 6 and 7.

Fig. 6 represents electrogram before AC IRE ablation, where records 1-2 are records of surface ECG; signals 3-7 are intracardial signals from ostia of pulmonary vein, signals 8-9 are intracardial signals from right chamber, record 10 is invasive measurement of blood pressure and detail 11 is detail of atrial potential of pulmonary vein recorded with circular catheter.

Fig. 7 represents electrogram after AC IRE ablation, where records 1-2 are records of surface ECG, signals 3-7 are intracardial signals from ostium of pulmonary vein; signals 8-9 are intracardial signals from right chamber, record 10 is invasive measurement of blood pressure and detail 11 is detail of disappearance of atrial potential from pulmonary vein after application of energy, which was recorded with circular catheter.

The functionality of electroporation generator according to the present invention is noticeable from observing details 11 in Figs. 6 and 7. Undesirable atrial potential from pulmonary vein recorded with circular catheter before intervention is in Fig. 6 in signals 3-7 clearly apparent, whereas in Fig. 7, that is after the intervention, is no longer apparent.

Great benefit of alternating electroporation generator according to the present invention is an extraordinary variability of settings of parameters of electroporation pulses:
- voltage up to thousands V,
- frequency of tens to hundreds of kHz,
- length of pulses of tens to hundreds of µs,
- lengths of spaces between pulses of hundreds ms to units of s or based on ECG signal,
- output currents of tens A.

An advantage of the present invention is, that the presence of pulse transformer on the output of the generator according to the present invention significantly increases operational safety of the source, from the perspective of protection against dangerous contact voltage, but mainly it does not, in principle, allow uncontrolled longtime or permanent transmission of direct voltage from direct power supply directly to output in the case of failure in power converter transistors, by which it significantly decreases the risk for the patient.

Alternating electroporation generator according to the present invention is also equipped with galvanically separated current sensor 13 of the output current into catheter 15 that is connected to display device 14 or to any measurement card. Since the pulse has constant alternating voltage, the quality of contact of application electrode with tissue may be judged from the magnitude of current response also during pulse application. Magnitude of the current amplitude and its time change during pulse application can reveal faults in generator or in application electrode, which is an important piece of information for the surgeon.

Alternation electroporation generator according to the present invention is intended as basic piece of electrophysiology lab within cardiology department.

## Claims

1. Alternating electroporation generator, **characterized in that,** it contains control unit (8) connected with regulation unit (9), direct current power supply (10), and power converter (11), containing storage capacitor and four power transistors of type MOSFET, connected into H-bridge, in whose diagonal is connected primary winding of the output high-frequency pulse transformer (12) on ferrite core, whose secondary winding is connected with at least one application electrode.

2. Alternating electroporation generator according to the claim 1, **characterized in that,** there is one or two application electrodes.

3. Alternating electroporation generator according to claims 1 to 2, **characterized in that,** the application electrodes are at least two catheters (15) with one contact area or at least one catheter (15) with more contact areas or catheter (15) in combination with large-area contact electrode (16) for placing in contact with body surface of the patient (17).

4. Alternating electroporation generator according to claims 1 to 3, **characterized in that,** pulse transformer (12) is preferably in the form of changeable module allowing its change for different type of transformer with different ratio between magnitude of output voltage and current.

5. Alternating electroporation generator according to any of claims 1 to 4, **characterized in that,** direct current power supply (10) is supplied by rectified mains voltage, whereas it is a changeable direct current power supply that enables setting of magnitude of the output pulse voltage.

6. Alternating electroporation generator according to any of claims 1 to 5, **characterized in that,** it further contains current sensor (13) on the output, that allows through display device (14) to display course of current of the electroporation pulse brought into application electrodes.

7. Alternating electroporation generator according to any of claims 1 to 6, **characterized in that,** control unit (8) is designed to control power converter (11), thereby enabling the setting of all parameters of the pulse created by it.

8. Alternating electroporation generator according to any of claims 1 to 7, **characterized in that,** it further comprises ECG sensing electrode (5) connected with ECG monitor (6) connected with block (7) of ECG analysis and synchronization of pulses connected with control unit (8).

9. Use of alternating electroporation generator according to any of claims 1 to 8 for cardiology.
